# EUROPÄISCHE PATENTANMELDUNG

(11) **EP 0 943 325 A1**
(43) Veröffentlichungstag der Anmeldung: **22.09.1999**
(21) Anmeldenummer: 98118760.2
(22) Anmeldetag: 05.10.1998
(51) Int. Cl.: A61K 9/00, A61K 9/16

(54) **Verfahren zur Herstellung homöopathischer Arzneimittel**

(30) Priorität: 18.03.1998 CH 64898
(71) Anmelder: Moser, Gerhard, 5000 Aarau (CH)
(72) Erfinder: Moser, Gerhard, 5000 Aarau (CH)
(74) Vertreter: Morva, Tibor

(57) **Zusammenfassung**

Nach dem Verfahren zur Herstellung homöopathischer Arzneimittel wird eine Ausgangssubstanz zuerst für eine Verdünnung vorbereitet. Nachher wird die Ausgangssubstanz mit einer neutralen Trägersubstanz im Verhältnis von eins zu Potenzen von zwei (1 : 2ⁿ) nach einem in der Homöopathie bekannten Verfahren stufenweise steigend mehrfach verdünnt. Der Exponent (n) ist dabei eine ganze Zahl, Null inbegriffen. Die in der Ausgangssubstanz vorhandenen Informationen bleiben bei diesem Verfahren bei der Potenzierung voll erhalten und auf die Trägersubstanz mit voller Wirksamkeit übertragen. Dieses Verfahren ermöglicht die Herstellung hochwirksamer homöopathischer Arzneimittel.

## Beschreibung

Die vorliegende Erfindung betrifft ein Verfahren zur Herstellung homöopathischer Arzneimittel mit mindestens einer Ausgangssubstanz und mit mindestens einer zur Verdünnung der Ausgangssubstanz dienenden Trägersubstanz.

Aus der DE-A1-3932100 ist ein Verfahren zur Herstellung homöopathischer Arzneimittel in Hochpotenzen bekannt. Bei diesem Verfahren erfolgt die Herstellung der homöopathischen Arzneimittel in Hochpotenzen, wie M-, XM-, XMII-, CH-, CMII und MM-Potenzen, indem man eine Gewichtsmenge einer C3-Verreibung einer zu potenzierenden Ausgangsubstanz in verdünntem Aethanol löst, davon einen Tropfen in 2,5 ml hochprozentigem Aethanol löst und 100mal kräftig schüttelt und mit dieser Lösung wenige Gramm Streukügelchen der Grösse I gleichmässig befeuchtet und diese in einem geschlossenen Gefäss an der Luft trocknet. Die hier aufgeführten Hochpotenzen, oder die Verhältnisse der stufenweisen Verdünnung der Ausgangsszbstanz richten sich nach den Potenzen von hundert, oder wie man in der Homöopathie üblicherweise schreibt, Cₙ. Die Wirksamkeit dieser Arzneimittel ist mindestens teilweise unbefriedigend.

Hahnemann, der Begründer der klassischen Homöopathie beschreibt in seinem Buch "Reine Arzneimittellehre" die Nutzung von Arzneimitteln bis zu einer Verdünnung von C₆₀, was eine Verdünnung von 1 : 100⁶⁰ bedeutet. Warum Hahnemann bei der Wahl der Verdünnungsgrade ausgerechnet bei einem Verhältnis von 1 : 100 angelangt ist, bleibt unklar. Selbst spätere Entwicklungen der Q-Potenzen (1 : 50000ⁿ) beruhen auf 100-er Potenzen. Warum Arzneimittel anscheinend nur auf mehr oder minder bestimmten Potenzstufen (Verdünnungsstufen) ihre Wirkung optimal entfalten, bleibt bisher im Dunkeln.

Die Aufgabe der vorliegenden Erfindung ist es, ein Verfahren zur Herstellung homöopathischer Arzneimittel zu entwickeln, das die Herstellung hochwirksamer homöopathischer Arzneimittel ermöglicht.

Die gestellte Aufgabe ist dadurch gelöst, dass die Ausgangssubstanz zuerst in eine für die nachfolgende Verdünnung geeignete Form gebracht wird und dann mit der neutralen Trägersubstanz im Verhältnis von eins zu Potenzen von zwei (1 : 2ⁿ) nach einem in der Homöopathie bekannten Verfahren stufenweise steigend mehrfach verdünnt wird, wobei der Exponent (n) eine ganze Zahl, Null inbegriffen, ist. Die stufenweise Verdünnung eines homöopathischen Arzneimittels nach den 2-er Potenzen durchzuführen, bedeutet in der Analogie zur Musik eine "Oktavierung" und somit die Einführung von O-Potenzen. Die Oktave ist sowohl mathematisch, technisch als auch musikalisch betrachtet ein Mass, welches bestimmte Verhältnisse regelt, die in einer festen Grössenordnung zueinander stehen. Diese Grössenordnungen sind bestimmt durch die Frequenz von Schwingungen, nämlich eine Verdoppelung oder eine Halbierung der Frequenz nach ganzzahligen Grössenänderungen. Die auf in Oktaven höher abgestimmten Resonanzkörper lassen sich durch die Grundfrequenz erregen. Aus der Literatur ist es bekannt, dass Flüssigkeiten, wie z.B. Wasser, als Flüssigkristall betrachtet werden können, die über eine bestimmte Gedächtnisfunktion verfügen. Diese Feststellung ist für die Entwicklung des Potenzierungsverfahrens nach 2-er Potenzen von entscheidender Bedeutung. Nach heutigen Erkenntnissen sind homöopathische Arzneimittel in höheren Verdünnungen wirksam. Es wird angenommen,dass die Wirksamkeit nur über die Uebertragung der wirksamen Information auf dem Wege der Frequenzübertragung geschieht. Eine Frequenzübertragung, bei der der Informationsgehalt der Welle nicht geändert werden soll, erfolgt idealerweise durch Oktavierung. Bei der qualitativen Verbesserung der Herstellung homöopathischer Arzneimittel wird davon ausgegangen, dass jede Ausgangssubstanz eine ihr eigene Frequenz besitzt, dass diese mitgetragene Information feststellbar ist, dass diese Informationen auf andere Träger übertragbar sind, dass diese Informationen an lebenden Organismen eine Reaktion bewirken können. Der Erhalt der vollständigen Informationen ist nur in einer Teilung bei den Verdünnungsstufen im Verhältnis von eins zu Potenzen von zwei gewährleistet, wobei der Exponent eine ganze Zahl, Null inbegriffen, ist.

Eine in der Trägersubstanz nichtlösliche Ausgangssubstanz wird vorteilhafterweise vor der Verdünnung zermahlen und anschliessend im Verhältnis von eins zu Potenzen von zwei (1 : 2ⁿ) nach Vorschriften des homöopathischen Arzneibuches mit der neutralen Trägersubstanz stufenweise steigend mehrfach verrieben, wobei der Exponent (n) eine ganze Zahl, Null inbegriffen, ist. Die Verreibung ist ein in der Homöopathie mit Erfolg verwendetes Verfahren. Die Verreibung erfolgt in Verreibungsstufen, indem man zur zermahlenen Ausgangssubstanz im Verhältnis von eins zu Zweierpotenzen eine neutrale Trägersubstanz zufügt. Zwischen den Verreibungsstufen liegen jeweils die Zermahlungsvorgänge.

Die in der Trägersubstanz nichtlösliche Ausgangssubstanz kann mit der neutralen Trägersubstanz mindestens bis zum Verhältnis von 1 : 2²⁰ verrieben werden, um die Löslichkeit der Ausgangssubstanz in der Trägersubstanz zu erreichen. Ueber diese Verdünnung sind alle festen Stoffe in einer flüssigen Trägersubstanz löslich.

Die in der Trägersubstanz nichtlösliche und mindestens im Verhältnis von 1 : 2²⁰ verriebene Ausgangssubstanz kann mit der neutralen Trägersubstanz im Verhältnis von eins zu Potenzen von zwei (1 : 2ⁿ) unter Schüttelschlägen stufenweise zu einer höheren Verdünnung weiter verdünnt werden. Nachdem über ein Verreibungsverhältnis von 1 : 2²⁰ alle festen Stoffe in einer flüssigen Trägersubstanz löslich sind, kann eine weitere Verdünnung der Ausgangssubstanz durch das einfachere, kostengünstigere Verschüttelungsverfahen erfolgen.

Eine in der Trägersubstanz lösliche Ausgangssubstanz wird vorteilhafterweise unter Schüttelschlägen mit der neutralen Trägersubstanz verdünnt. Dies ist ein in der Homöopathie angewendetes, einfaches Verfahren. Um die Vermischung der Substanzen miteinander optimal durchzuführen, sollten in jeder Verdünnungsstufe mindestens 10 Schüttelschläge durchgeführt werden.

Das Verhältnis der Verdünnung wird mit Vorteil mindestens bis 1 : 2³⁰ durchgeführt, um die Grenze der toxikologischen Unbedenklichkeit zu erreichen. Ueber diese Verdünnungsgrenze sind toxikologische Wirkungen praktisch ausgeschlossen.

Das Verhältnis der Verdünnung kann vorteilhafterweise mindestens bis 1 : 2⁸⁰ durchgeführt werden, um die materielle Grenze zu erreichen. Bei diesem Verdünnungsverhältnis erreicht man die sogenannte materielle Grenze, bei welcher nicht einmal ein Molekül der Ausgangssubstanz im homöopathischen Arzneimittel vorhanden sein dürfte, wenn die Ausganssubstanz ein Mol war. Die Zahl 2⁸⁰ liegt nämlich über der Avogadro'schen oder Loschmidt'schen Zahl 0,6023 ×10²⁴.

Im folgenden werden Ausführungsbeispiele der Erfindung näher beschrieben.

Die Verdünnungen im Verhältnis von 1 : 2ⁿ werden in den folgenden Beispielen als O-Potenzen bezeichnet, eine Oₙ Verdünnung bedeutet demnach ein Verdünnungsverhältnis von 1 : 2ⁿ .

Die Abkürzung HAB bedeutet "Homöopathisches Arzneibuch" und GT "Gewichtsteil".

### Beispiel 1

### (Verreibung)

Die in gängigen Flüssigkeiten nichtlösliche Ausgangssubstanz, Schwefel wird in einem Mörser mit der Trägersubstanz, Milchzucker im Gewichtsverhältnis von 1 : 1 zermahlen. Die vorgeschriebene Körnung des Endergebnisses richtet sich nach den Vorschriften des HAB. Bei dem Gewichtsverhältnis 1 : 1 sind Messungenauigkeiten weitgehendst vermeidbar. Allerdings soll das Gewichtsverhältnis von 1 : 1 dieser ersten Verfahrensstufe bei Explosivstoffen, wie Nitroglycerin oder bei hoch toxischen Substanzen, wie Botulintoxin nicht unbedingt eingehalten werden.

Die Verfahrensschritte sind die folgenden:
1 GT Schwefel + 1 GT Milchzucker, Verreibung = O₁
1 GT O₁ Schwefel + 1 GT Milchzucker, Verreibung = O₂
1 GT O₂ Schwefel + 1 GT Milchzucker, Verreibung = O₃
1 GT O₃ Schwefel + 1 GT Milchzucker, Verreibung = O₄
1 GT O₄ Schwefel + 1 GT MIlchzucke usw.

### Beispiel 2

### (Verschüttelung)

Die in gänglichen Flüssigkeiten lösliche Ausgangssubstanz, der Pflanzenauszug Belladonnatinktur wird mit einer alkoholischen Lösung verdünnt. Die alkoholische Lösung besteht aus 40 GT Wasser und 60 GT Alkohol. Für andere, fettlösliche Ausgangssubstanzen wird vorzugsweise Glycerin oder eine in der medizinischen Arzneimittelherstellung übliche Lösungsmittel verwendet.

Die Verfahrensschritte sind die folgenden:
1 GT Belladonnatinktur + 1 GT alkoholische Lösung, Verschüttelung = O₁
1 GT O₁ Belladonnatinktur + 1 GT alkoholische Lösung, Verschüttelung = O₂
1 GT O₂ Belladonnatinktur + 1 GT alkoholische Lösung, Verschüttelung = O₃
1 GT O₃ Belladonnatinktur + 1 GT alkoholische Lösung, usw.

Um eine ausreichende Vermischung der Ausgangssubstanz mit der Trägersubstanz zu erreichen, werden in jeder Verdünnungsstufe mindestens 10 Schüttelschläge durchgeführt.

### Beispiel 3

### (gemischtes Herstellungsverfahren)

Die Verfahrensschritte sind die folgenden:
1 GT Schwefel + 1 GT Milchzucker, Verreibung = O₁
1 GT O₁ Schwefel -- 1 GT Milchzucker, Verreibung = O₂
1 GT O₂ Schwefel + 1 GT Milchzucker, Verreibung = O₃

Diese Reihe wird fortgesetzt, bis die Verreibung O₂₀ erreicht ist. Es ist nämlich bekannt, dass über die Verreibung O₂₀ alle in der Trägersubstanz anfänglich nichtlöslichen Ausgangssubstanze lösbar werden. Deshalb kann die weitere Verdünnung der Ausgangssubstanz mit einer alkoholischen Lösung mit 40 GT Wasser und 60 GT Alkohol durch eine einfache Verschüttelung ausgeführt werden. Die weiteren Verfahrensschritte sind:
1 GT O₂₀ Schwefel + 1 GT alkoholische Lösung, Verschüttelung = O₂₁
1 GT O₂₁ Schwefel + 1 GT alkoholische Lösung, Verschüttelung = O₂₂
1 GT O₂₂ Schwefel -- 1 GT alkoholische Lösung, Verschüttelung = O₂₃
1 GT O₂₃ Schwefel -- 1 GT alkoholische Lösung, usw.

Die Verreibungsstufen werden in einem Mörser durch Verreibung durchgeführt. Zwischen den einzelnen Verschüttelungsstufen werden je mindestens 10 Schüttelschläge ausgeführt.

Die Reihe der Verfahrensschritte wird bis zur Verschüttelung = O₃₀ fortgesetzt. Das dadurch gegebene Verdünnungsverhältnis von 1 : 2³⁰ ist die Grenze der toxikologischen Unbedenklichkeit. Ueber diese Verdünnungsgrenze dürften toxikologische Wirkungen praktisch ausgeschlossen sein.

Anschliessend wird die Verdünnung bis zur Verschüttelung = O₈₀ weitergeführt. Bei diesem Verdünnungsverhältnis von 1 : 2⁸⁰ erreicht man die sogenannte materielle Grenze, bei welcher nicht einmal ein Molekül der Ausgangssubstanz im homöopathischen Arzneimittel vorhanden sein dürfte, sofern der Ausgangs substanz ein Mol war. Die Zahl 2⁸⁰ liegt nämlich über der Avogadro'schen oder Loschnidt'schen Zahl 0.6023×10²⁴.

Um die auf die Trägersubstanz übertragene Informationen oder Schwingungen der Ausgangssubstanz gegen Fremdeinflüsse zu schützen, wird das durch das Herstellungsverfahren gewonnene Arzneimittel in einem als Faraday'scher Käfig dienenen Metallbehälter, vorzugsweise mit einer Bleischicht oder in einer lichtdichten Verpackung, wie Blauglas aufbewahrt. Die wesentlichsten, die Informationen oder Schwingungen im hergestellten homöopathischen Arzneimittel möglicherweise beeinflussenden Fremdeinflüsse sind hochfrequente elektromagnetische Felder, Alpha-, Beta- und Gammastrahlen.

Das erfindungsgemäss hergestellte homöopathische Arzneimittel kann in allen üblichen Applikationsarten verabreicht werden. Die hohe Wirksamkeit des nach O-Potenzen potenzierten homöopathischen Arzneimittels wurde in der Praxis beobachtet.

## Patentansprüche

1. Verfahren zur Herstellung homöopathischer Arzneimittel mit mindestens einer Ausgangssubstanz und mit mindestens einer zur Verdünnung der Ausgangssubstanz dienenden Trägersubstanz, **dadurch gekennzeichnet, dass** die Ausgangssubstanz zuerst in eine für die nachfolgende Verdünnung geeignete Form gebracht wird und dann mit der neutralen Trägersubstanz im Verhältnis von eins zu Potenzen von zwei (1 : 2ⁿ) nach einem in der Homöopathie bekannten Verfahren stufenweise steigend mehrfach verdünnt wird, wobei der Exponent (n) eine ganze Zahl, Null inbegriffen, ist.

2. Verfahren nach Anspruch 1, **dadurch gekennzeichnet, dass** eine in der Trägersubstanz nichtlösliche Ausgangssubstanz vor der Verdünnung zermahlen und anschliessend im Verhältnis von eins zu Potenzen von zwei (1 : 2ⁿ) nach Vorschriften des homöopathischen Arzneibuches mit der neutralen Trägersubstanz stufenweise steigend mehrfach verrieben wird, wobei der Exponent (n) eine ganze Zahl, Null inbegriffen, ist.

3. Verfahren nach Anspruch 2, **dadurch gekennzeichnet, dass** die in der Trägersubstanz nichtlösliche Ausgangssubstanz mit der neutralen Trägersubstanz mindestens bis zum Verhältnis von 1 : 2²⁰ verrieben wird, um die Löslichkeit der Ausgangssubstanz in der Trägersubstanz zu erreichen.

4. Verfahren nach Anspruch 3, **dadurch gekennzeichnet, dass** die in der Trägersubstanz nichtlösliche und mindestens im Verhältnis von 1 : 2²⁰ verriebene Ausgangssubstanz mit der neutralen Trägersubstanz im Verhältnis von eins zu Potenzen von zwei (1 : 2ⁿ) unter Schüttelschlägen stufenweise zu einer höheren Verdünnung weiter verdünnt wird.

5. Verfahren nach Anspruch 1, **dadurch gekennzeichnet, dass** eine in der Trägersubstanz lösliche Ausgangssubstanz unter Schüttelschlägen mit der neutralen Trägersubstanz verdünnt wird.

6. Verfahren nach den Ansprüchen 4 oder 5, **dadurch gekennzeichnet, dass** in jeder Verdünnungsstufe mindestens 10 Schüttelschläge durchgeführt werden.

7. Verfahren nach einem der Ansprüche 1, 4, **dadurch gekennzeichnet, dass** das Verhältnis der Verdünnung mindestens bis 1 : 2³⁰ durchgeführt wird, um die Grenze der toxikologischen Unbedenklichkeit zu erreichen.

8. Verfahren nach einem der Ansprüche 1, 4, **dadurch gekennzeichnet, dass** das Verhältnis der Verdünnung mindestens bis 1 : 2⁸⁰ durchgeführt wird, um die materielle Grenze zu erreichen.
